(19) Europäisches Patentamt
European Patent Office
Office européen des brevets

(11)     EP 2 643 022 B1

(12)                     **EUROPEAN PATENT SPECIFICATION**

(45) Date of publication and mention
of the grant of the patent:
**25.07.2018 Bulletin 2018/30**

(21) Application number: **11790937.4**

(22) Date of filing: **24.11.2011**

(51) Int Cl.:
*A61K 47/69* (2017.01)          *A61K 31/352* (2006.01)
*A61K 9/20* (2006.01)          *A61K 9/00* (2006.01)

(86) International application number:
**PCT/EP2011/070951**

(87) International publication number:
**WO 2012/069591 (31.05.2012 Gazette 2012/22)**

(54) **FAST DISINTEGRATING COMPOSITIONS COMPRISING NABILONE AND RANDOMLY METHYLATED BETA CYCLODEXTRIN**

SCHNELL ZERFALLENDE ZUSAMMENSETZUNGEN MIT NABILON UND ZUFÄLLIG METHYLIERTEM BETA-CYCLODEXTRIN

COMPOSITIONS À DÉLITEMENT RAPIDE COMPRENANT DE LA NABILONE ET DE LA BÊTA-CYCLODEXTRINE MÉTHYLÉE DE MANIÈRE ALÉATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.11.2010 EP 10192497**

(43) Date of publication of application:
**02.10.2013 Bulletin 2013/40**

(73) Proprietor: **AOP Orphan Pharmaceuticals AG**
**1160 Wien (AT)**

(72) Inventors:
• **VIERNSTEIN, Helmut**
**A-1180 Wien (AT)**
• **TOEGEL, Stefan**
**A-3013 Tullnerbach (AT)**
• **SCHUELLER, Regina**
**A-1080 Wien (AT)**

(74) Representative: **Loidl, Manuela Bettina et al**
**REDL Life Science Patent Attorneys**
**Donau-City-Straße 11**
**1220 Wien (AT)**

• **SOUTER REX W.: "Nabilone", ANALYTICAL PROFILES OF DRUG SUBSTANCES, vol. 10, 1981, pages 499-512, XP008134537,**
• **"Martindale - The complete drug reference (32nd Edition), Nabilone" In: 1999, MARTINDALE : THE COMPLETE DRUG REFERENCE. (FORMERLY MARTINDALE THE EXTRA PHARMACOPOEIA), LONDON : PHARMACEUTICAL PRESS, GB, PAGE 1203, XP002629156, ISBN: 978-0-85369-429-8 the whole document**
• **SZEJTLI J ED - SZEJTLI J: "CYCLODEXTRINS IN PHARMACEUTICALS", 1 January 1988 (1988-01-01), CYCLODEXTRIN TECHNOLOGY, DORDRECHT, KLUWER, NL, PAGE(S) 186 - 307, XP001194813, * page 289, section 3.4 "Cyclodextrins as Auxiliary Substances" ***
• **HAZEKAMP A ET AL: "Structure elucidation of the tetrahydrocannabinol complex with randomly methylated beta-cyclodextrin", EUROPEAN JOURNAL OF PHARMACEUTICAL SCIENCES, ELSEVIER, AMSTERDAM, NL, vol. 29, no. 5, 1 December 2006 (2006-12-01), pages 340-347, XP025137176, ISSN: 0928-0987, DOI: DOI:10.1016/J.EJPS.2006.07.001 [retrieved on 2006-12-01] cited in the application**

(56) References cited:
**WO-A1-03/070774     US-A1- 2011 028 431**

- MANNILA J ET AL: "Effects of RM-beta-CD on sublingual bioavailability of DELTA<9>-tetrahydrocannabinol in rabbits", EUROPEAN JOURNAL OF PHARMACEUTICAL SCIENCES, ELSEVIER, AMSTERDAM, NL, vol. 26, no. 1, 1 September 2005 (2005-09-01), pages 71-77, XP025316273, ISSN: 0928-0987, DOI: DOI:10.1016/J.EJPS.2005.04.020 [retrieved on 2005-09-01]

- "CAVASOL W7 M Pharma", Wacker - Fine Chemicals , XP002629767, Retrieved from the Internet: URL:http://www.cyclodextrin.org/CDPDF/CS_W 7_M_P.pdf [retrieved on 2011-03-24]

**Description**

The present invention provides a novel composition comprising Nabilone and randomly methylated ß-cyclodextrin (RAMEB), wherein the weight ratio (dry weight to dry weight) between Nabilone and RAMEB is 1:60 - 1:140.

Nabilone is a fully synthetic and crystalline cannabinoid with therapeutic use as an antiemetic and anti-anxiety agent and as an adjunct analgesic for neuropathic pain. Nabilone was first approved in 1985 in the US under the trade name "Cesamet" in the form of rigid gelatin capsules. The positive effect of using Nabilone for the treatment of chemotherapy-induced nausea and vomiting (CINV) and increase of the life quality of patients was shown in several clinical studies.

The aqueous solubility of Nabilone is extremely low, less than 0.5 μg/ml at 25°C. The occurrence of at least four distinct polymorphic forms with different bioavailability characteristics further complicates the development of a stable dosage form.

Until present, due to its poor solubility in water, Nabilone is available only as gelatin capsule which is highly disadvantageous especially for patients suffering from nausea who have difficulties to swallow these capsules.

Cyclodextrins are well established as pharmaceutical excipients forming host-guest complexes with hydrophobic molecules, thereby increasing water solubility of the guest molecule. Cyclodextrins are cyclic oligosaccharides consisting of (α-1,4) linked α-D-glucopyranose units, with a lipophilic central cavity and a hydrophilic outer surface. Typical cyclodextrins are constituted by 6-8 glucopyranoside units and can be topologically represented as toroids with the larger and the smaller openings of the toroid exposing to the solvent secondary and primary hydroxyl groups respectively. Due to this arrangement, the interior of the toroids is not hydrophobic, but considerably less hydrophilic than the aqueous environment and thus able to host other hydrophobic molecules. In contrast, the exterior is sufficiently hydrophilic to convert the water solubility of the cyclodextrins or their complexes.

The formation of the inclusion compounds greatly modifies the physical and chemical properties of the guest molecule, mostly in terms of water solubility.

In WO03/070774 the use of various cyclodextrins for increasing the solubility of classical, naturally occurring cannabinoids isolated from Cannabis has been described. Haazekamp A and Verpoorte R. describe solubility testing of classical naturally occurring cannabinoid Tetrahydrocannabinoid in the presence of various cyclodextrins (European J. of Pharm Sciences 29 (2006), 340-347).

Souter R., "Nabilone", Analytical Profiles of Drug Substances, vol. 10, 1981, 499-512 describes the physical-chemical characteristics of Nabilone.

Szejtli J., "Cyclodextrins in Pharmaceuticals", 1988, Cyclodextrin Technology, 186-307 describes the use of cyclodextrin as auxiliary substance.

Mannila J. et al.,Europ.J.Pharm.Sciences, 26 (2005), 71-77 disclose sublingual formulations of cannabinoids.

Any increase of solubility of synthetic cannabinoids was not disclosed.

The object of the present invention is to provide Nabilone in a formulation which can overcome the disadvantages as listed above.

The object is solved by the embodiments of the present invention.

Brief description of the invention:

The present invention provides a formulation preferably for a fast disintegrating administration form like, for example, a tablet, containing Nabilone wherein Nabilone has increased solubility in aqueous solutions and increased stability. The better solubility can thus overcome the high first-pass metabolism and low dissolution properties.

The inventive composition specifically comprises Nabilone and randomly methylated ß-cyclodextrin (RAMEB), wherein the weight ratio (dry weight to dry weight) between Nabilone and RAMEB is about 1:60 - 1:140, preferably the weight ratio is about 1:90 - 1:110. Specifically, the inventive composition comprises Nabilone and randomly methylated ß-cyclodextrin (RAMEB) in the weight ratio (dry weight to dry weight) of 1:60 - 1:140, preferably in the weight ratio of 1:90 - 1:110, wherein Nabilone and RAMEB are comprised as an aqueous soluble complex. It has been surprisingly shown that the solubility of Nabilone was highly increased when RAMEB was used as complex-forming agent, being present in the composition in the above mentioned range.

According to an embodiment of the invention, Nabilone is present in the composition in an amount of 0.01 to 100mg, preferably of 0.1 to 50mg, more preferred of 0.25 to 40mg, more preferred in an amount of approx. 30mg with regard to the medication form.

The composition of the invention can further comprise at least one pharmaceutically acceptable carrier, adjuvant or additive or mixtures thereof. Specifically, the additive is a disintegrating agent. More specifically, the disintegrating agent is selected from microcrystalline cellulose, starches, sodium starch glycolate, croscarmelose sodium, crospovidone, povidone, calcium silicate.

According to a further embodiment of the invention the composition comprises a pharmaceutically acceptable carrier selected from the group of magnesium stearate, magnesium fumarate, sodium hydrogen carbonate, citric acid anhydride, talc, sorbitol, mannitol, carboxymethylcellulose, lactose, hydroxypropylmethylcellulose, collidone or carbopol.

According to a specific embodiment, Nabilone and RAMEB are comprised as lyophilized complex in the composition. Alternatively, Nabilone and RAMEB may also be comprised as inclusion body complex, non-inclusion body complex or as co-precipitate. Non-complexed Nabilone may further be comprised in the inventive composition. More specifically, the composition comprises a complex of Nabilone and RAMEB together with sodium-hydrogen carbonate, citric acid anhydride and/or crospovidone, wherein Nabilone and RAMEB are present in the weight ratio (dry weight to dry weight) of 1:60 - 1:140, preferably in the weight ratio of 1:90 - 1:110.

The composition of the invention can be in any form useful for administration of Nabilone, specifically it can be a tablet, a capsule, a spray, a solution or a chewing gum. Even more specific, the solution comprises about 30 weight% RAMEB.

Oral, preferably sublingual or buccal, are the preferred administration routes of the inventive composition.

Fast disintegrating tablets (FDT) are preferred administration forms as they rapidly disintegrate and/or dissolve to release the drug as soon as they get into contact with saliva, thus obviating the need for water during administration, an attribute that makes them highly attractive for patients experiencing difficulty in swallowing tablets such as patients with persistent nausea. Additionally, absorption can result in improved bioavailability and as a result of reduced dosage, improved clinical performance through a reduction of unwanted effects.

According to a specific embodiment of the invention, a FDT is provided which comprises between 10 and 15 wt%, preferably about 11.5 wt% Nabilone-RAMEB complex, between 60 and 95 wt%, preferably about 87.5 wt% disintegrating agent and between 0.5 and 5 wt%, preferably about 1 wt% pharmaceutically acceptable carrier.

The inventive composition can be used as a medicament. Specifically it can be used for the prevention or treatment of nausea, muscular spasm, multiple sclerosis, uterine and bowel cramps, movement disorders, pain, including migraine headache, glaucoma, asthma, inflammation, insomnia, high blood pressure and/or a condition responsive to an appetite stimulating, amyotrophic lateral sclerosis anti-cancer, oxytoxic, anxiolytic, anti-convulsive, anti-depressant and anti-psychotic agent.

The present invention further provides a method for producing a complex of RAMEB and Nabilone, wherein Nabilone and RAMEB are present in the weight ratio (dry weight to dry weight) of 1:60 - 1:140, preferably in the weight ratio of 1:90 - 1:110 and wherein said RAMEB is combined with Nabilone in a heterogeneous state or in a solid state, including using methods selected from freeze drying, spray-drying, kneading, grinding, slurry-method, co-precipitation, and neutralization, and optionally separating the obtained complex.

Specifically, the method for producing aqueous soluble Nabilone comprises the step of complexing Nabilone with RAMEB.

Further, a method for increasing the bioavailability of Nabilone from a sublingually or buccally administered preparation is provided comprising the step of complexing Nabilone with RAMEB and forming the complex so obtained into a sublingually or buccally administered dosage form.

A method for increasing the solubility of Nabilone in aqueous solution by complexing Nabilone and RAMEB is also provided by the present invention wherein Nabilone is stirred for about 96 h in the presence of about 25 % or 30% RAMEB and maintaining a constant reaction temperature of about 25°C.

**Figures:**

Fig. 1: Chemical structure of Nabilone
Fig. 2: Standard curve of Nabilone
Fig. 3: Standard curve of the Nabilone-RAMEB complex
Fig. 4: The solubility of Nabilone in water as a function of RAMEB concentration at 25°C.
Fig. 5: The solubility of Nabilone in RAMEB solutions as a function of reaction time.
Fig. 6: The solubility of Nabilone in RAMEB solutions (5-50%) at 25°C.
Fig. 7: The solubility of Nabilone in RAMEB solutions after 96 h reaction time at different reaction temperatures.
Fig. 8: Solubility isotherms of the Nabilone-RAMEB complex after 96 h reaction time at different reaction temperatures.

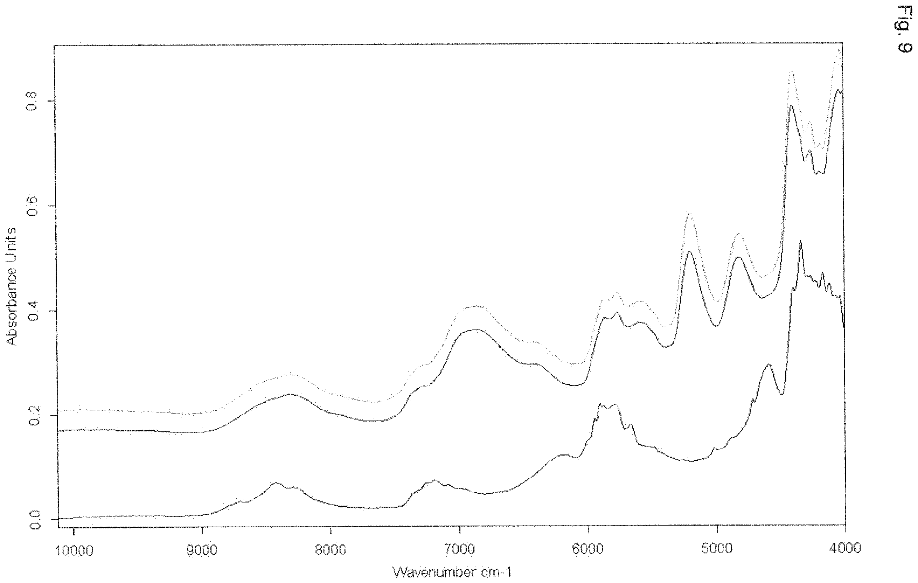

Fig. 9: The NIR spectra of Nabilone (lower line), RAMEB (upper line), and the Nabilone-RAMEB complex (middle line).

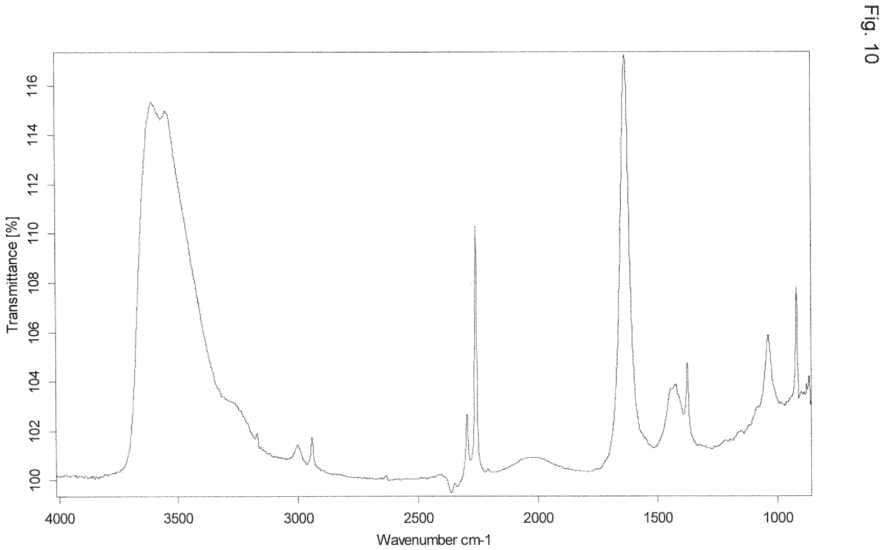

Fig. 10: The FTIR spectra of Nabilone.

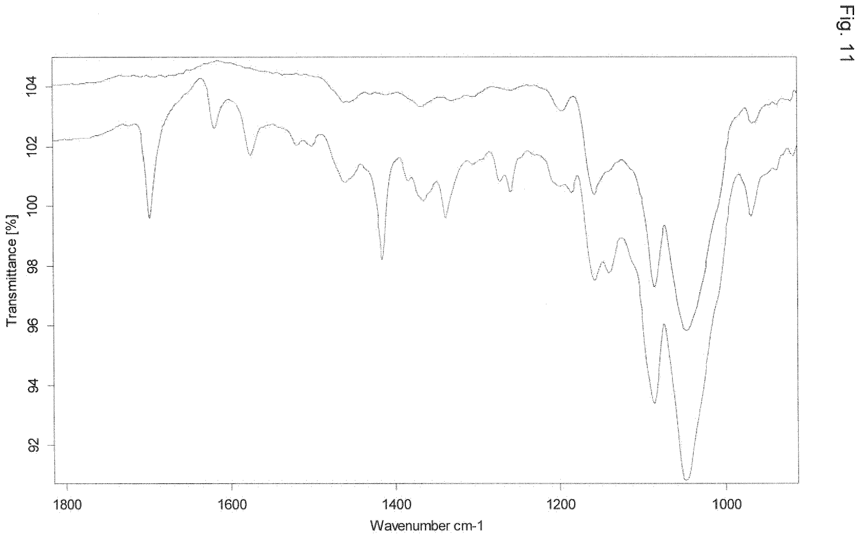

Fig. 11: The FTIR spectra of RAMEB (upper line) and the Nabilone-RAMEB complex (lower line) at a range between 1,800 and 900 $cm^{-1}$.

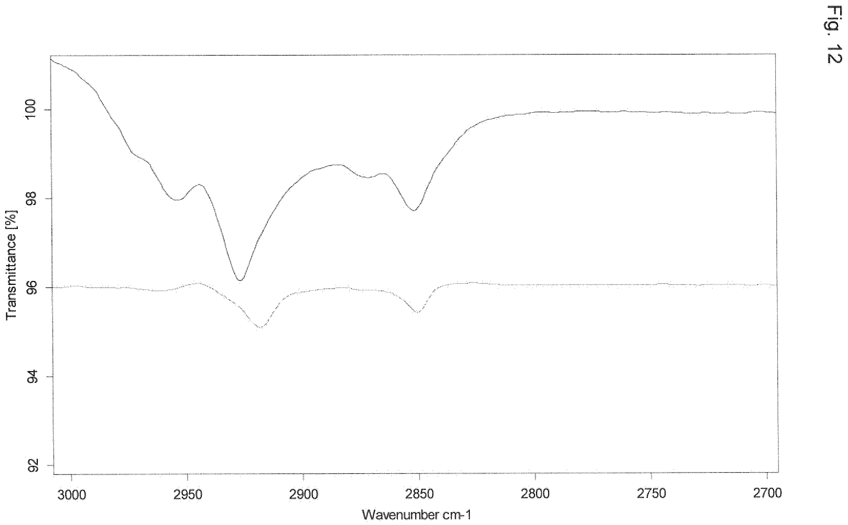

Fig. 12: The FTIR spectra of RAMEB (lower line) and the Nabilone-RAMEB complex (upper line) at a range between 3,000 and 2,700 $cm^{-1}$.

**EP 2 643 022 B1**

Detailed description of the invention

The present invention covers a composition comprising Nabilone and randomly methylated ß-cyclodextrin (RAMEB) in the weight ratio (dry weight to dry weight) of 1:60 - 1:140, preferably in the weight ratio of 1:90 - 1:110 wherein Nabilone and RAMEB are comprised as an aqueous soluble complex. As an alternative embodiment, the composition may further comprise additional non-complexed Nabilone, specifically in the range of about 0.001 - 0.01:60, more specifically of about 0.001 - 0.01:140.

Alternatively, the invention provides a composition comprising Nabilone and RAMEB, wherein the weight ratio (dry weight to dry weight) between Nabilone and RAMEB is about 1:60 to 1:140, preferably the weight ratio is about 1:90 to 1:110.

According to the present invention the term "complex" denotes an inclusion body complex, a non-inclusion body complex, a co-precipitate or a lyophilized complex. Preferably, it is a lyophilized complex or an inclusion complex. It was surprisingly found that the presence of RAMEB in the specific range of weight ratios can increase the aqueous solubility of Nabilone significantly. The use of RAMEB as complex-forming agent in the specific concentration range is thus supposed to be essential for the remarkable increase of the aqueous solubility of Nabilone. It was also shown by the present invention that other cyclodextrins which are, according to the prior art, equivalent as solubility enhancers did not show the significant effect on the solubility of Nabilone. The inclusion of Nabilone into RAMEB results in much higher affinity than with unmethylated ß-CD or with the trimethylated compound. The selection of RAMEB is essential for the present invention.

Thus, high concentrations of Nabilone in aqueous solutions can be achieved. The most preferred weight ratio (dry weight to dry weight) is between 1:60 and 1:140, preferably between 1:90 and 1:110. When RAMEB is used in an aqueous solution, such solution preferably contains RAMEB in a concentration of about 20 to 35%, preferably about 25 to 30% by weight. RAMEB in a concentration of 40 weight% or more results in a decrease in Nabilone solubility. A formulation for a fast disintegrating tablet comprising Nabilone can further be provided having Nabilone-RAMEB complexes as active principle, thus providing increased solubility of Nabilone in aqueous solutions and preventing instability of the drug associated with polymorphic forms. Specifically, said complexes are inclusion and/or non-inclusion complexes.

The inventive composition can further contain pharmaceutically acceptable carriers, adjuvants or additives as known in the art. The additive can for example be a disintegrating agent. Disintegrating agents are known in the art, for example Ludiflash® is a well known accelerator for providing fast disintegrating administration forms. Ludiflash® comprises mannitol, crospovidone, povidone and polyvinylpyrrolidone.

Specifically for making oral administration forms like fast disintegrating tablets (FDT) or capsules, disintegrating agents can be used, specifically agents like microcrystalline cellulose, starches, sodium starch glycolate, croscarmelose sodium, polyvinylpyrrolidone, povidone or calcium silicate or combinations thereof.

Further pharmaceutically acceptable carriers like magnesium stearate, magnesium fumarate, sodium hydrogen carbonate, citric acid anhydride, talc, sorbitol, mannitol, carboxymethylcellulose, lactose, hydroxypropylmethylcellulose, collidone and carbopol can be present in the inventive composition.

As well known in the art additional further ingredients can also be added to improve the taste of the oral administration forms.

More specifically, the Nabilone/RAMEB-complex composition may comprise additional agents like sodium-hydrogen carbonate, citric acid anhydride and crospovidone and combinations thereof.

According to an embodiment of the present invention, a FDT is provided comprising between 10 and 15 wt% Nabilone-RAMEB complex, between 60 and 95 wt% disintegrating agent and between 0.5 and 5 wt% pharmaceutically acceptable carrier.

More specifically, the FDT formulation comprises about 11.5% Nabilone/RAMEB complex, about 87.5% disintegrating agent and about 1% pharmaceutically acceptable carrier. Any disintegrating agent and carrier material and any combination thereof may be used therefore.

According to a specific embodiment, the disintegrating agent is Ludiflash® and/or crosscarmelose and the pharmaceutically acceptable carrier is sodium hydrogen carbonate citric acid anhydride and/or talc.

A specific composition may contain Nabilone and randomly methylated ß-cyclodextrin (RAMEB) in the weight ratio (dry weight to dry weight) of 1:60 - 1:140, preferably in the weight ratio of 1:90 - 1:110 wherein Nabilone and RAMEB are comprised as an aqueous soluble complex, sodium-hydrogen carbonate, citric acid anhydride and crospovidone or any combinations thereof. As a further embodiment, non-complexed Nabilone may additionally be comprised in said composition.

Additionally, mucoadhesive polymers for buccal delivery such as agarose, chitosan, gelatin, hyaluronic acid, various gums, cellulose derivates like CMC, thiolated CMC, HEC, HPC, HPMC; polyacrylates, methacrylates, polyoxyethylene, PVA, thiolated polymers or combinations thereof can also be comprised in the inventive composition.

The inventive composition can be in any form useful for administering Nabilone, specifically it can be in the form of a tablet, for example a bioadhesive tablet for buccal delivery, a capsule, a spray, a solution or a chewing gum.

Alternatively, also topical administration of the composition is provided, like creams, ointments, jellies, solutions or suspensions wherein the composition is formulated together with a pharmaceutically acceptable carrier. Alternatively, also pulmonary or nasal administration forms can be provided.

If the complex is in form of a solution, the solution preferably comprises about 30 weight% RAMEB. When using RAMEB as complex forming agent, it has been shown that solutions comprising between 25 and 35%, specifically $\geq 25$ and $\leq 35\%$, preferably about 30% RAMEB lead to highly increased aqueous solubility of Nabilone.

The composition can be a pharmaceutical composition used as medicament, specifically for oral use, more specifically for sublingual or buccal use.

When tablets are the selected administration form, the present invention also provides an optimized method for production of fast disintegrating tablets wherein the compression force is optimizing hardness and disintegration time of said tablets containing Nabilone which may increase the optimal availability of the active agent. The compression force is of about 1.2 kN and appears specifically advantageous for the production of fast disintegrating tablets containing the inventive Nabilone-RAMEB complex.

The therapeutic dose of Nabilone may vary depending on the age and body weight of the individual as well as the particular condition to be treated and the manner of administration. The dose can easily be determined by a skilled person.

According to a specific embodiment, the amount of Nabilone present in the composition for oral use can be 0.01 mg to 5g, preferably 0.01 mg to 100mg, preferably of 0.1 and 50mg, more preferred of 0.25 and 15mg, more preferred in an amount of about 0.3mg. The composition can be given several times a day as applicable.

Due to the increased bioavailability of the inventive Nabilone-RAMEB complex composition and by overcoming the loss of active agent due to the first-pass metabolism, smaller dosages of Nabilone can also be administered.

The inventive composition can be used for the treatment of any disease wherein administering a synthetic cannabinoid is applicable. For example it can be used for the treatment of nausea, muscular spasm, multiple sclerosis, uterine and bowel cramps, movement disorders, pain, including migraine headache, glaucoma, asthma, inflammation, insomnia, high blood pressure and/or for the treatment of a condition responsive to an appetite stimulating, amyotrophic lateral sclerosis, anti-cancer, oxytoxic, anxiolytic, anti-convulsive, anti-depressant and anti-psychotic agent.

The present invention also provides a method for producing a complex of RAMEB and Nabilone, wherein Nabilone and RAMEB are present in the weight ratio (dry weight to dry weight) of 1:60 - 1:140, preferably in the weight ratio of 1:90 - 1:110 and wherein said RAMEB is combined with Nabilone in a heterogeneous state or in the solid state by using methods selected from freeze drying, spray-drying, kneading, grinding, slurry-method, co-precipitation, and neutralization, and optionally separating the obtained complex.

By lyophilizing a solution containing RAMEB and Nabilone, Nabilone can be provided as homogenous powder having excellent dissolution properties. In this powder, Nabilone is complexed with RAMEB forming inclusion bodies. The dissolution rate of Nabilone is increased due to the excellent solubility and dissolution properties of RAMEB.

Thus, the present invention provides a method for producing aqueous soluble Nabilone comprising the step of complexing Nabilone with RAMEB.

A method for increasing the bioavailability of Nabilone in a sublingually or buccally administered preparation is also provided comprising the steps of complexing Nabilone with RAMEB and formulating the complex obtained as a sublingually or buccally administerable dosage form.

As an alternative embodiment the present invention further provides a method for increasing the solubility of the Nabilone in aqueous solution by complexing Nabilone and RAMEB wherein Nabilone is stirred for about 96 h in the presence of about 25% to about 30 weight% RAMEB and maintaining a constant reaction temperature of about 25°C.

The examples described herein are illustrative of the present invention and are not intended to be limitations thereof. Different embodiments of the present invention have been described according to the present invention. Many modifications and variations may be made to the techniques described and illustrated herein without departing from the spirit and scope of the invention. Accordingly, it should be understood that the examples are illustrative only and are not limiting upon the scope of the invention.

Examples:

**Materials**

Nabilone was obtained from Loba Feinchemie (Fischamend, Austria). Gamma-Cyclodextrin ($\gamma$-CD; Gamma W8 Pharma), hydroxypropyl-ß-Cyclodextrin (HP-ß-CD; Cavasol W7 HP Pharma) and randomly methylated β-Cydodextrin (RAMEB; Cavasol® W7 M) were purchased from Wacker Chemie (Munich, Germany). Alpha-Cyclodextrin (a-CD; Cavamax W6) and beta-Cyclodextrin (ß-CD; Kleptose) were obtained from International Specialty Products Inc (Cologne, Germany) and Roquette ((Lestrem, France), respectively. ß-CD sulfobutyl ether sodium salt (SBE-ß-CD; Captisol) was provided by Cydex Pharmaceuticals (Lenexa, USA). For HPLC, bidistilled water was prepared using a Buchi Fontavapor 285 (Essen, Germany) whereas acetonitrile was obtained from Sigma (Vienna, Austria). For the preparation of FDT formulation,

Ludiflash® was obtained from BASF (Vienna, Austria). Cross-linked Sodium Carboxymethyl Cellulose (Na-CMC, Croscarmellose,Croscarmelose sodium®) was obtained from FMC (Brussels, Belgium). Sodium hydrogen carbonate and citric acid anhydride were obtained from Kwizda (Vienna, Austria).

## Methods

*Preparation of Nabilone - cyclodextrin inclusion complexes*

For the preparation of solubility isotherms, a surplus of Nabilone was added to a dilution series of cyclodextrins in distilled water. The surplus of Nabilone was approximately 130% of the maximum amount of Nabilone that could be resolved theoretically in the cyclodextrin solution. For the preparation of standard curves, an accurately weighted amount of Nabilone was added to an aqueous RAMEB solution. Then, the suspensions were stirred at 600 rpm at a constant temperature (4°C - 40°C) in the cold room or in an environmental simulation chamber for the time periods indicated. Afterwards, the samples were centrifuged at 10,000 rpm and filtrated to remove undissolved particles prior to freeze-drying or HPLC analysis.

*Lyophilization of Nabilone - cyclodextrin inclusion complexes*

The filtrated solutions of the Nabilone - cyclodextrin complexes were frozen as a thin film on the inner side of round bottom glass container in a methanol - dry ice bath. Lyophilization was performed for 20 h using a Heto Power Dry LL3000 instrument (Thermo Fisher Scientific).

*Estimation of the Nabilone content in the lyophilisates*

Freeze-dried products were stirred with acetonitrile for 30 min to promote complex dissociation and extraction of Nabilone. Samples were centrifuged and the residue was washed again two times with acetonitrile. Combined acetonitrile supernatants were evaporated under vacuum and the residue dissolved in a defined amount of acetonitrile. After centrifugation (10,000 rpm, 5 min) the content of Nabilone was measured using HPLC and related to 1 ml of the initial reaction solution.

*Analysis of the Nabilone - RAMEB complex*

*HPLC*

HPLC analysis of the Nabilone-RAMEB complex was performed according to standard procedures.

*Near-infrared spectroscopy (NIR)*

Near-infrared spectra in the 12.500-4.000 $cm^{-1}$ region were obtained using a Bruker MPA FT-NIR spectrometer (Bruker Optics Inc). Measurements were performed by inserting the Fibre Optic Module into powdered samples of Nabilone, RAMEB, or the lyophilized Nabilone-RAMEB complex.

*Fourier transform infrared spectroscopy (FTIR)*

FTIR spectra were measured with a Bruker Tensor 27 FTIR instrument (Bruker Optics, Billerica, MA) equipped with a liquid nitrogen cooled mercury-cadmium-telluride (MCT) detector. 2% aqueous solutions of RAMEB and Nabilone-RAMEB complex as well as a 0.1% solution of Nabilone in acetonitrile were prepared and 20 $\mu$l of each sample were loaded into the BioATR II cell. Spectral scans in the range of 900-4,000$cm^{-1}$ were obtained and corrected by subtraction of the solvent spectra as background. Data were collected and analyzed using the OPUS software provided with the instrument.

*Molecular modeling*

Initially, the centre of mass of Nabilone was inserted into the centre of mass of the ß-CD molecule, followed by MM+ optimization of the inclusion complex using the HyperChem software package. This preliminary structure was used as starting point for the geometry optimization of the Nabilone-CD complex by a more advanced DFT (Density Functional Theory) method (B3LYP/6-31G(d,p)) implemented in the GAUSSIAN03 program package. Both isolated molecules of the complex (Nabilone and β-CD were also optimized by this method and the differences of the energies were calculated to obtain interaction energies according to the following equation:

$$E_{int} = E_{complex} - E_{CD} - E_{Nabilone}$$

*Preparation of an FDT formulation containing the Nabilone-RAMEB complex*

*Lyophilization of the Nabilone-RAMEB complex*

A 30% RAMEB solution was stirred with a surplus of Nabilone at 25°C as described previously. After filtration of the suspension through a 0.22 μm membrane, the filtrate was frozen using dry ice and methanol. Lyophilization was performed as described above and the content of complexed Nabilone was determined by HPLC.

*Formulation*

Tablets with defined weights were prepared by directly compressing formulations containing 30mg lyophilized RAMEB or Nabilone-RAMEB complex, 1% talk (w/w, based on the tablet mass) and different amounts of Ludiflash® (Table 1). The components were admixed lege artis and the tablets were pressed with a Korsch EK0 instrument (Instrumentation by Hottinger Baldwin Messtechnik- DCM plus, Software: BEAM). 500mg was chosen as the upper limit of the tablet weight in order to follow the FDA guidelines for "Orally Disintegrating Tablets", which recommend that the weight of such tablets should not exceed 500mg. In addition, some 260mg tablets were prepared replacing 10% of Ludiflash® with Croscarmellose sodium® or a mixture of sodium hydrogen carbonate and citric acid anhydride. Moreover, the compression force was varied in order to produce tablets with different hardness characteristics.

Table 1:

| Composition | | | | |
|---|---|---|---|---|
| Tablet weight (mg) | Lyophilized RAMEB or Nabilone-RAMEB complex (mg) | Ludiflash® (mg) | Talk (mg) | Other excipients (mg) |
| 200 | 30 | 168 | 2 | - |
| 260 | 30 | 227.5 | 2.5 | - |
| 400 | 30 | 366 | 4 | - |
| Composition | | | | |
| Tablet weight (mg) | Lyophilized RAMEB or Nabilone-RAMEB complex (mg) | Ludiflash® (mg) | Talk (mg) | Other excipients (mg) |
| 500 | 30 | 465 | 5 | - |
| 260 | 30 | 204.4 | 2.6 | 23 Croscarmellose sodium® |
| 260 | 30 | 204.4 | 2.6 | 23 Na- hydrogen carbonate & citric acid anhydride (1:1) |

*FDT characteristics*

*Hardness*

Hardness of the tablets was controlled using a Pharma Test PTB 311 apparatus (Hainburg, Germany).

*Disintegration*

The FDA describes the "Orally Disintegrating Tablets" as solid oral preparations that disintegrate rapidly in the oral cavity, with an in-vitro disintegration time of approximately 30 seconds or less, based on the conventional USP disintegration test method. This method, however, does not appear always appropriate for testing FDTs and cannot adequately reflect in-vivo conditions. Thus, the USP disintegration method has been modified as follows. Disintegration was monitored by submerging the tablets into 2 ml of distilled water at 25°C or 37°C. In order to simulate the oral cavity conditions, the samples were shaken horizontally at 40 rpm using an Inova 4000 shaker (New Jersey, USA). The time necessary for complete disintegration of tablets was recorded.

*Dissolution*

For monitoring the dissolution of Nabilone from the FDT formulation, an amount of Nabilone-RAMEB complex corresponding to the content in a tablet charge was weighted. Both the weighted complex and the FDT were solubilized in 1 ml water and the content of Nabilone in the supernatant was quantified using HPLC.

Results

*HPLC analysis of Nabilone in acetonitrile*

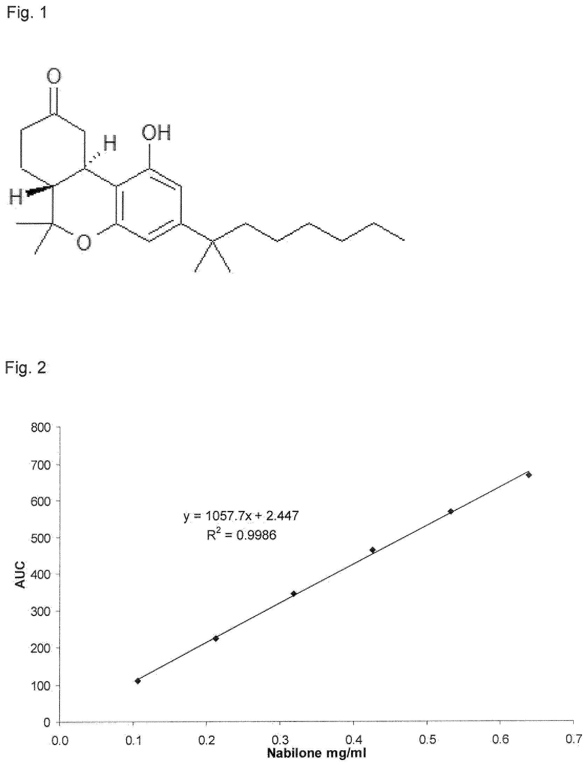

A dilution series of accurately weighted Nabilone in acetonitrile was analyzed using HPLC. Figure 2 shows the resulting standard curve.

*Comparison of different Nabilone-cyclodextrin complexes*

Many cyclodextrins and cyclodextrin derivatives are not soluble in acetonitrile, which in contrast is the optimal solvent for Nabilone, preventing analysis of the Nabilone-cyclodextrin complexes with HPLC. In order to directly compare different cyclodextrins regarding their complexation capacity for Nabilone, it has been decided to freeze-dry the cyclodextrin-Nabilone complexes and, afterwards, extract the drug from the complex using acetonitrile.

Table 2 shows the amount of Nabilone ($\mu$g/ml) that was solubilized by 1 ml of 6 different cyclodextrins. The concentrations of the cyclodextrin solutions were adapted to their solubility in water. Whenever possible, concentrations of 15%, 20%, and 30% were selected. In general, most of the studied cyclodextrin solutions, except for that of $\gamma$-CD and SBE-ß-CD, increased the solubility of Nabilone when compared to water (<0.5 $\mu$g/ml). Whereas ß-CD increased the Nabilone solubility about 4-fold (2.3$\pm$0.3 $\mu$g/ml at 1.5%), the use of $\alpha$-CD resulted in a 30-fold increase (17.1$\pm$1.3 $\mu$g/ml at 12%). In contrast, 1 ml of 30% HP-ß-CD solubilized 44.5$\pm$0.8 $\mu$g Nabilone (90-fold increase). Of note, a remarkable increase of the aqueous solubility of Nabilone was achieved using RAMEB as complex-forming agent. Aqueous RAMEB solutions dose dependently increased the Nabilone solubility up to 9,000-fold as determined using this protocol, resulting in 4,460$\pm$180 $\mu$g/ml Nabilone at 30% RAMEB.

Based on these experiments, RAMEB was selected as solubility enhancing agent for further studies.

Table 2. The amount of Nabilone solubilized by 1 ml of various cyclodextrin solutions as determined by extraction of the drug from lyophilized complexes.

| | | Nabilone ($\mu$g/ml) |
|---|---|---|
| **H$_2$O** | | 0.5 |
| **RAMEB** | 15% | 2620$\pm$70 |
| | 20% | 3330$\pm$390 |
| | 30% | 4460$\pm$180 |
| **HP-ß-CD** | 15% | 16.2$\pm$5.4 |
| | 20% | 32.2$\pm$0.1 |
| | 30% | 44.5$\pm$0.8 |
| **$\alpha$-CD** | 8% | 15.3$\pm$0.4 |
| | 10% | 13.8$\pm$7.7 |
| | 12% | 17.1$\pm$1.3 |
| **ß-CD** | 1% | 2.0$\pm$0.4 |
| | 1.25% | 2.8$\pm$0.6 |
| | 1.50% | 2.3$\pm$0.3 |
| **SBE-ß-CD** | 30% | < 1 |
| **$\gamma$-CD** | 20% | < 1 |

*Standard curve of the Nabilone-RAMEB complex*

Beside the remarkable complex formation capacity of RAMEB with Nabilone, RAMEB is also beneficial regarding its solubility in acetonitrile, allowing direct HPLC analysis of the complex.

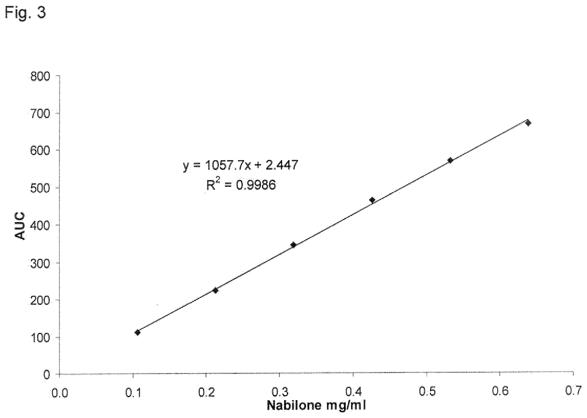

Figure 3 shows a dilution series of the Nabilone-RAMEB complex, analyzed using HPLC. Measurements above 1mg/ml Nabilone were found to exceed the range of linearity, indicating the necessity to dilute samples of the Nabilone-RAMEB complex below 1mg/ml.

Of note, the similarity of the data in Figure 2 and Figure 3 indicates that the HPLC results of the Nabilone-RAMEB complex indeed result from the inclusion of Nabilone into the cavity of RAMEB.

*Solubility isotherms of the Nabilone-RAMEB complex*

In contrast to the other studied cyclodextrins, RAMEB markedly increased the aqueous solubility of Nabilone and allowed direct HPLC analysis of the complex without the detour of lyophilization and solvent extraction.

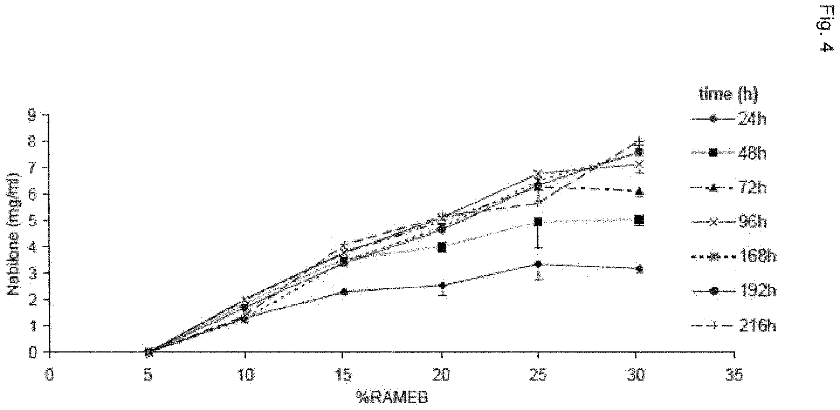

Figure 4 shows the increase in the solubility of Nabilone with increasing concentrations of RAMEB after various time periods for complexation at 25°C. From the solubility isotherm after 96 h, a solubility constant of K = 270,000 was calculated. Based on the solubility of Nabilone in water (0.5 $\mu$g/ml), the inclusion of Nabilone into RAMEB (30%, 92 h) resulted in >10,000-fold increased solubility values (7.14mg/ml).

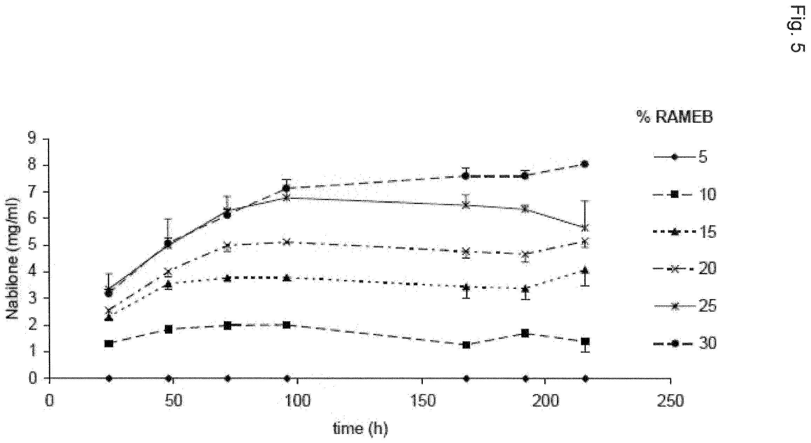

Figure 5 presents the data as a function of the reaction time. After 4 days of stirring, the inclusion process appears finished, since no increase of the Nabilone solubility was observed afterwards.

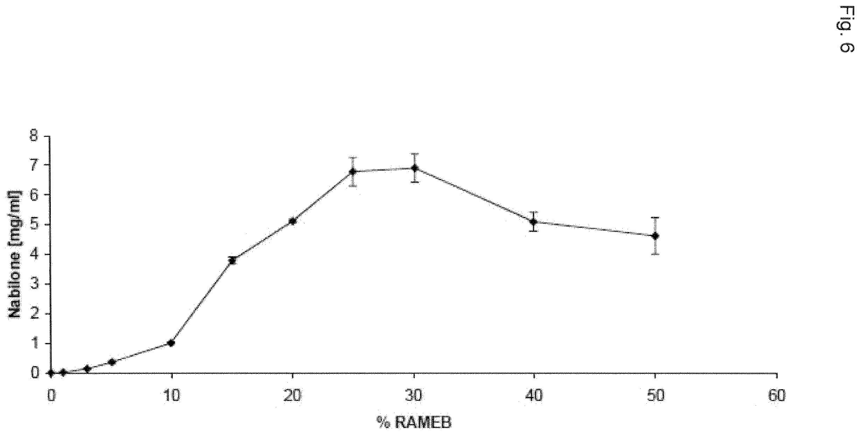

Figure 6 indicates that no further increase of Nabilone solubility was yielded using RAMEB concentrations above 30%. In contrast, using 40% or 50% RAMEB caused a slight decrease in Nabilone solubility.

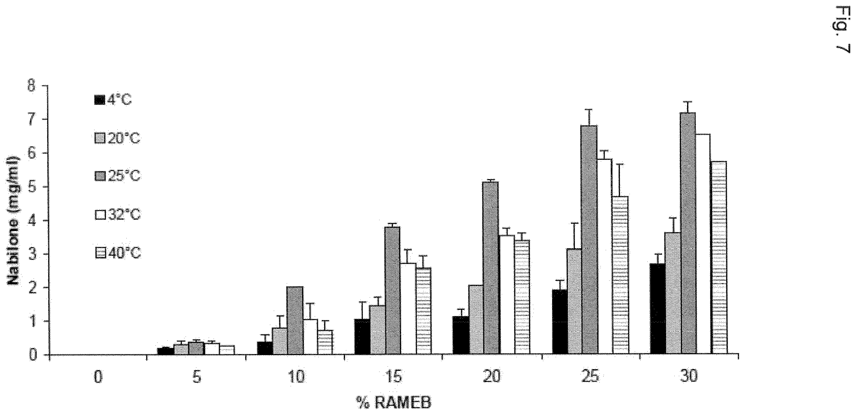

Figure 7 shows the influence of reaction temperature on the complex formation between RAMEB and Nabilone. The results clearly indicate that a reaction temperature of 25°C induced the highest increase of Nabilone solubility in RAMEB solutions at all tested concentrations. Of note, the Nabilone-RAMEB complex formation significantly decreased at reaction temperatures above 25°C, i.e. 32°C or 40°C.

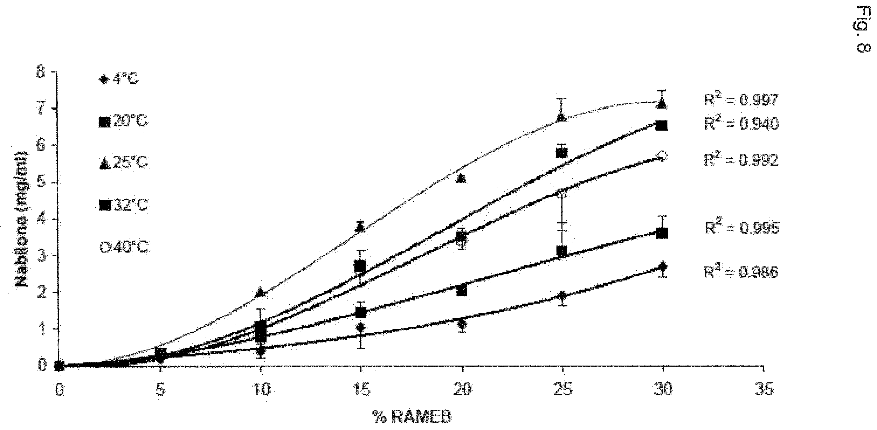

Figure 8 shows the solubility isotherms of the Nabilone-RAMEB complex at 5 different reaction temperatures. The solubility isotherms are characterized by a slight sigmoid shape and a high coefficient of determination ($R^2$=0.940 - 0.997).

*FDT characteristics*

*Effect of the amount of Ludiflash® on FDT characteristics*

The previous experiments have shown that - in order to provide a single dose of 0.3mg Nabilone - about 30mg of the Nabilone-RAMEB complex should be used for the preparation of 1 FDT dosage form. It was found that the addition of RAMEB to any FDT formulation results in significantly increased disintegration times. For the preparation of FDTs, Ludiflash® was used as a ready excipient containing mannitol, soluble binder and super-disintegrant agent. In order to optimize the ratio between Ludiflash® and RAMEB used for an FDT formulation, the effect of increasing amounts of Ludiflash® on the hardness and disintegration time of FDTs containing 30mg of lyophilized RAMEB was investigated. For this purpose, tablets were compressed with the compression force of 1.2 kN. As a result, increasing amounts of Ludiflash® significantly decreased tablet hardness and disintegration time of tablets. Although the 500mg tablets disintegrated faster than those with 260mg, the later were selected for further developments since tablets with ≥ 400mg appeared impractical.

At this point, it appears necessary to remember that the disintegration time as monitored using the described in-vitro method unlikely reflects the actual disintegration time in-vivo. In preliminary in-vivo experiments it has been observed that disintegration time of 260mg FDTs containing 30mg RAMEB are significantly decreased as compared to the in-vitro method.

*Selection of the appropriate compression force for 260mg tablets*

260mg tablets containing 30mg lyophilized RAMEB, 2.6mg talk and 227.5mg Ludiflash® were prepared using different compression forces. Although the tablets compressed with 0.5 kN showed disintegration times of 60 s, it is assumed that their hardness (< 15 N) might not be adequate for packaging processes and administration. Taken together, a compression force of 1.2 kN appears feasible for the production of FDTs containing Nabilone-RAMEB complex.

*Impact of super-disintegrants on FDT characteristics*

In order to further improve the disintegration time of 260mg tablets, the influence of additional super-disintegrants and effervescent agents was investigated. 10% Ludiflash® were replaced by Croscarmellose sodium® or a blend of Na-hydrogen carbonate and citric acid anhydride. Tablets were pressed using 1.2 kN compression force.

*Dissolution of Nabilone from FDT*

As indicated in Table 4 no difference between the dissolution of Nabilone from the complex and the FDT was observed, suggesting that the used FDT excipients do not interfere with the dissolution of Nabilone.

Table 4. Dissolution of Nabilone from the Nabilone-RAMEB complex and from an FDT formulation containing Ludiflash® and the complex.

| Dissolution of Nabilone from | Test 1 (mg/ml) | Test 2 (mg/ml) |
|---|---|---|
| the complex | 0.166 | 0.220 |
| the FDT formulation | 0.176 | 0.216 |

**Conclusion**

In conclusion, the approach of enhancing the water solubility of Nabilone by forming inclusion complexes with cyclodextrins appears promising. At this, the use of RAMEB is preferable to the use of other cyclodextrins such as $\alpha$-CD, ß-CD, $\gamma$-CD, HP-ß-CD, or SBE-ß-CD. Optimized complexation conditions include (1) the use of 25% or 30% RAMEB, (2) stirring with Nabilone for 96 h, and (3) maintaining a constant reaction temperature of 25°C. Thereby, the solubility of the Nabilone in aqueous solution can be increased >10,000-fold, as compared to the extremely low solubility of Nabilone in water (0.5 $\mu$g/ml). Furthermore, the Nabilone-RAMEB complex can be easily processed into FDTs. In order to provide a single dose of 0.3mg Nabilone, it can be expected to use about 30mg of the Nabilone-RAMEB complex for the preparation of 1 FDT dosage form. Since the addition of RAMEB to the FDT interferes with the speed of disintegration, the composition of the formulation was optimized as well as the compression force for tableting. One feasible formulation includes (1) 30mg of the Nabilone-RAMEB complex, (2) 1% talc, (3) 23mg of a blend of Na-hydrogen carbonate and citric acid anhydride and (4) Ludiflash® ad 100%, compressed with 1.2 kN into 260mg FDTs. These manufacturing conditions were shown to provide fast disintegration of the FDT and complete dissolution of the complex in aqueous body fluids. It is expected that the use of Nabilone-RAMEB complex in FDT dosage forms will favorably affect the stability as well as the bioavailability of Nabilone.

**Claims**

1. Composition comprising Nabilone and randomly methylated ß cyclodextrin (RAMEB) in the weight ratio (dry weight to dry weight) of 1:60 - 1:140, preferably in the weight ratio of 1:90 - 1:110 and wherein Nabilone and RAMEB are comprised as an aqueous soluble complex.

2. Composition according to claim 1, wherein Nabilone is present in an amount of 0.01 to 100mg, preferably of 0.1 to 50mg, more preferred of 0.25 to 10mg, more preferred in an amount of 30mg.

3. Composition according to claims 1 or 2 wherein said composition additionally comprises non-complexed Nabilone.

4. Composition according to any one of claims 1 to 3 further comprising an additive, wherein the additive is a disintegrating agent, specifically selected from microcrystalline cellulose, starches, sodium starch glycolate, crosscarmelose sodium, crospovidone, povidone, calcium silicate.

5. Composition according to any one of claims 1 to 4 comprising a pharmaceutically acceptable carrier selected from the group of magnesium stearate, magnesium fumarate, sodium hydrogen carbonate, citric acid anhydride, talc, sorbitol, mannitol, carboxymethylcellulose, lactose, hydroxypropylmethylcellulose, collidone, carbopol.

6. Composition according to any one of claims 1 to 5 wherein the solution comprises RAMEB in a concentration of 25% to 30% by weight.

EP 2 643 022 B1

7. Composition according to any one of claims 1 to 6 wherein Nabilone and RAMEB are comprised as lyophilized complex.

8. Composition according to any one of claims 1 to 7, comprising a complex of Nabilone and RAMEB, sodium-hydrogen carbonate, citric acid anhydride and crospovidone, wherein Nabilone and RAMEB are present in the weight ratio (dry weight to dry weight) of 1:60 - 1:140, preferably in the weight ratio of 1:90 - 1:110.

9. Composition according to any one of claims 1 to 8 for use as a medicament.

10. Composition according to any one of claims 1 to 9 for oral, preferably for sublingual or buccal use.

11. Composition according to any one of claims 1 to 10 for use in the treatment of nausea, muscular spasm, multiple sclerosis, uterine and bowel cramps, movement disorders, pain, including migraine headache, glaucoma, asthma, inflammation, insomnia, high blood pressure and/or a condition responsive to an appetite stimulating, amyotrophic lateral sclerosis, anti-cancer, oxytoxic, anxiolytic, anti-convulsive, anti-depressant and anti-psychotic agent.

12. Method for producing a complex of RAMEB and Nabilone, wherein Nabilone and RAMEB are present in the weight ratio (dry weight to dry weight) of 1:60 - 1:140, preferably in the weight ratio of 1:90 - 1:110 and wherein said RAMEB is combined with Nabilone in a heterogeneous state or in the solid state, including using methods selected from freeze drying, spray-drying, kneading, grinding, slurry-method, co-precipitation, and neutralization, and optionally separating the obtained complex.

13. Method for producing aqueous soluble Nabilone according to claim 12 comprising the step of complexing Nabilone with RAMEB.

14. Method for increasing the bioavailability of Nabilone in a sublingually or buccally administered preparation comprising the step of complexing Nabilone with RAMEB according to claim 12 or 13 and formulating the complex obtained as a sublingually or buccally administerable dosage form.

15. Method for increasing the solubility of the Nabilone in aqueous solution by complexing Nabilone and RAMEB according to claim 12 or 13, wherein Nabilone is stirred for about 96 h in the presence of 25% or 30 weight % RAMEB and maintaining a constant reaction temperature of about 25°C.

16. Composition according to any one of claims 1 to 11 comprising between 10 and 15 wt%, preferably about 11.5 wt% Nabilone-RAMEB complex, between 60 and 95 wt%, preferably about 87.5 wt%, disintegrating agent and between 0.5 and 5 wt%, preferably about 1 wt% pharmaceutically acceptable carrier.


**Patentansprüche**

1. Zusammensetzung, umfassend Nabilon und methyliertes ß-Cyclodextrin (RAMEB, vom engl. randomly methylated ß cyclodextrin) im Gewichtsverhältnis (Trockengewicht/Trockengewicht) von 1:60 - 1:140, bevorzugt im Gewichtsverhältnis von 1:90 - 1:110, und wobei Nabilon und RAMEB als ein wasserlöslicher Komplex umfasst sind.

2. Zusammensetzung nach Anspruch 1, wobei Nabilon in einer Menge von 0,01 bis 100 mg, bevorzugt von 0,1 bis 50 mg, stärker bevorzugt von 0,25 bis 10 mg, stärker bevorzugt in einer Menge von 30 mg vorhanden ist.

3. Zusammensetzung nach Anspruch 1 oder 2, wobei die Zusammensetzung zusätzlich nicht-komplexiertes Nabilon umfasst.

4. Zusammensetzung nach einem der Ansprüche 1 bis 3, ferner umfassend einen Zusatz, wobei der Zusatz ein Sprengmittel ist, welches spezifisch aus mikrokristalliner Cellulose, Stärken, Natriumstärkeglykolat, Croscarmellose-Natrium, Crospovidon, Povidon und Calciumsilikat ausgewählt ist.

5. Zusammensetzung nach einem der Ansprüche 1 bis 4, umfassend einen pharmazeutisch verträglichen Träger, welcher aus der Gruppe aus Magnesiumstearat, Magnesiumfumarat, Natriumhydrogencarbonat, Zitronensäureanhydrid, Talk, Sorbitol, Mannitol, Carboxymethylcellulose, Laktose, Hydroxymethylcellulose, Collidon und Carbopol ausgewählt ist.

**6.** Zusammensetzung nach einem der Ansprüche 1 bis 5, wobei die Lösung RAMEB in einer Konzentration von 25 bis 30 Gewichts-% umfasst.

**7.** Zusammensetzung nach einem der Ansprüche 1 bis 6, wobei Nabilon und RAMEB als lyophilisierter Komplex umfasst sind.

**8.** Zusammensetzung nach einem der Ansprüche 1 bis 7, umfassend einen Komplex aus Nabilon und RAMEB, Natriumhydrogencarbonat, Zitronensäureanhydrid und Crospovidon, wobei Nabilon und RAMEB im Gewichtsverhältnis (Trockengewicht/Trockengewicht) von 1:60 - 1:140, bevorzugt im Gewichtsverhältnis von 1:90 - 1:110 vorhanden sind.

**9.** Zusammensetzung nach einem der Ansprüche 1 bis 8 zur Verwendung als ein Medikament.

**10.** Zusammensetzung nach einem der Ansprüche 1 bis 9 zur oralen, bevorzugt zur sublingualen oder bukkalen Anwendung.

**11.** Zusammensetzung nach einem der Ansprüche 1 bis 10 zur Verwendung bei der Behandlung von Übelkeit, Muskelkrampf, Multipler Sklerose, Gebärmutter- und Darmkrämpfen, Bewegungsstörungen, Schmerzen, einschließlich Migränekopfschmerz, Glaukom, Asthma, Entzündung, Schlaflosigkeit, Bluthochdruck und/oder einem Zustand infolge eines appetitanregenden, amyothrophischen Lateralsklerose-, Krebs-, oxytoxischen, anxiolytischen, antikonvulsiven, Antidepressions- und antipsychotischen Mittels.

**12.** Verfahren zum Herstellen eines Komplexes aus RAMEB und Nabilon, wobei Nabilon und RAMEB im Gewichtsverhältnis (Trockengewicht/Trockengewicht) von 1:60 - 1:140, bevorzugt im Gewichtsverhältnis von 1:90 - 1:110 vorhanden sind, und wobei das RAMEB mit Nabilon in einem heterogenen Zustand oder im festen Zustand kombiniert wird, einschließlich der Verwendung von Verfahren, welche aus Gefriertrocknen, Sprühtrocknen, Kneten, Mahlen, Aufschlämmverfahren, Kopräzipitation und Neutralisation und optional Trennen des erhaltenen Komplexes ausgewählt sind.

**13.** Verfahren zum Herstellen von wasserlöslichem Nabilon nach Anspruch 12, umfassend den Schritt des Komplexierens von Nabilon mit RAMEB.

**14.** Verfahren zum Erhöhen der Bioverfügbarkeit von Nabilon in einem sublingual oder bukkal verabreichten Präparat, umfassend den Schritt des Komplexierens von Nabilon mit RAMEB gemäß Anspruch 12 oder 13 und des Formulierens des erhaltenen Komplexes als eine sublingual oder bukkal verabreichbare Dosierungsform.

**15.** Verfahren zum Erhöhen der Löslichkeit des Nabilons in wässriger Lösung durch Komplexieren von Nabilon und RAMEB gemäß Anspruch 12 oder 13, wobei Nabilon etwa 96 h in Gegenwart von 25 oder 30 Gewichts-% RAMEB gerührt und bei einer konstanten Reaktionstemperatur von etwa 25 °C gehalten wird.

**16.** Zusammensetzung nach einem der Ansprüche 1 bis 11, umfassend zwischen 10 und 15 Gew.-%, bevorzugt etwa 11,5 Gew.-% Nabilon-RAMEB-Komplex, zwischen 60 und 95 Gew.-%, bevorzugt etwa 87,5 Gew.-% Sprengmittel und zwischen 0,5 und 5 Gew.-%, bevorzugt etwa 1 Gew.-% pharmazeutisch verträglichen Träger.

**Revendications**

**1.** Composition comprenant de la nabilone et de la β-cyclodextrine méthylée de manière aléatoire(RAMEB) dans le rapport massique (poids sec sur poids sec) de 1:60 à 1:40, de préférence dans le rapport massique de 1:90 à 1:110 et ladite nabilone et ledit RAMEB étant constitués sous la forme d'un complexe soluble dans l'eau.

**2.** Composition selon la revendication 1, ladite nabilone étant présente en une quantité de 0,01 à 100 mg, de préférence de 0,1 à 50 mg, plus préférentiellement de 0,25 à 10 mg, plus préférentiellement en une quantité de 30 mg.

**3.** Composition selon la revendication 1 ou 2, ladite composition comprenant en outre de la nabilone non complexée.

**4.** Composition selon l'une quelconque des revendications 1 à 3, comprenant en outre un additif, ledit additif étant un agent délitant, choisi plus particulièrement parmi la cellulose microcristalline, les amidons, le glycolate d'amidon sodique, la crosscarmellose sodique, la crospovidone, la povidone, le silicate de calcium.

5. Composition selon l'une quelconque des revendications 1 à 4 comprenant un vecteur pharmaceutiquement acceptable choisi dans le groupe du stéarate de magnésium, du fumarate de magnésium, de l'hydrogénocarbonate de sodium, de l'anhydride d'acide citrique, du talc, du sorbitol, du mannitol, de la carboxyméthylcellulose, du lactose, de l'hydropropylméthylcellulose, de la collidone, du carbopol.

6. Composition selon l'une quelconque des revendications 1 à 5, ladite solution comprenant du RAMEB en une concentration de 25 % à 30 % en poids.

7. Composition selon l'une quelconque des revendications 1 à 6, ladite nabilone et ledit RAMEB étant constitués sous la forme d'un complexe lyophilisé.

8. Composition selon l'une quelconque des revendications 1 à 7, comprenant un complexe de nabilone et de RAMEB, de l'hydrogénocarbonate de sodium, de l'anhydride d'acide citrique et de la crospovidone, ladite nabilone et ledit RAMEB étant présents dans le rapport massique (poids sec sur poids sec) de 1:60 à 1:140, de préférence dans le rapport massique de 1:90 à 1:110.

9. Composition selon l'une quelconque des revendications 1 à 8 pour utilisation comme médicament.

10. Composition selon l'une quelconque des revendications 1 à 9 pour utilisation orale, de préférence pour utilisation sublinguale ou buccale.

11. Composition selon l'une quelconque des revendications 1 à 10 pour utilisation dans le traitement de la nausée, d'un spasme musculaire, de la sclérose en plaques, des crampes utérines et abdominales, des troubles moteurs, de la douleur, notamment la céphalée migraineuse, un glaucome, l'asthme, une inflammation, l'insomnie, une pression artérielle élevée et/ou un état sensible à une stimulation de l'appétit, la sclérose latérale amyotrophique, un agent anticancer, ocytocique, anxiolytique, anticonvulsif, antidépresseur et antipsychotique.

12. Procédé de production d'un complexe de RAMEB et de nabilone, ladite nabilone et ledit RAMEB étant présents dans le rapport massique (poids sec sur poids sec) de 1:60 à 1:140, de préférence dans le rapport massique de 1:90 à 1:110 et ledit RAMEB étant combiné à la nabilone dans un état hétérogène ou à l'état solide, notamment à l'aide des méthodes choisies parmi la lyophilisation, le séchage par pulvérisation, le malaxage, le broyage, un procédé en pâte, la co-précipitation et la neutralisation, et éventuellement la séparation du complexe obtenu.

13. Procédé de production de nabilone soluble dans l'eau selon la revendication 12 comprenant l'étape de complexation de la nabilone avec le RAMEB.

14. Procédé pour augmenter la biodisponibilité de la nabilone dans une préparation administrée par voie sublinguale ou buccale comprenant l'étape de complexation de la nabilone avec le RAMEB selon la revendication 12 ou 13 et de formulation du complexe obtenu sous une forme posologique administrable par voie sublinguale ou buccale.

15. Procédé pour augmenter la solubilité de la nabilone en solution aqueuse par complexation de la nabilone et du RAMEB selon la revendication 12 ou 13, ladite nabilone étant agitée pendant environ 96 h en présence de 25 % ou 30 % en poids de RAMEB en maintenant une température de réaction constante d'environ 25°C.

16. Composition selon l'une quelconque des revendications 1 à 11 comprenant entre 10 et 15 % en poids, de préférence environ 11,5 % en poids de complexe nabilone-RAMEB, entre 60 et 95 % en poids, de préférence environ 87,5 % en poids, d'un agent délitant et entre 0,5 et 5 % en poids, de préférence environ 1 % en poids d'un vecteur pharmaceutiquement acceptable.

Fig. 1

Fig. 2

$$y = 1057.7x + 2.447$$
$$R^2 = 0.9986$$

Fig. 3

$$y = 1057.7x + 2.447$$
$$R^2 = 0.9986$$

Fig. 4

Fig. 5

Fig. 6

Fig. 7

Fig. 8

Fig. 9

Fig. 10

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03070774 A

**Non-patent literature cited in the description**

- *European J. of Pharm Sciences,* 2006, vol. 29, 340-347
- **SOUTER R.** Nabilone. *Analytical Profiles of Drug Substances,* 1981, vol. 10, 499-512
- **SZEJTLI J.** Cyclodextrins in Pharmaceuticals. *Cyclodextrin Technology,* 1988, 186-307
- **MANNILA J. et al.** *Europ.J.Pharm.Sciences,* 2005, vol. 26, 71-77